# EUROPEAN PATENT APPLICATION

(11) **EP 4 781 957 A1**
(43) Date of publication of application: **29.07.2026**
(21) Application number: 25166022.1
(22) Date of filing: 25.03.2025
(51) Int. Cl.: A61F 9/007

(54) **AQUEOUS DRAINAGE IMPLANT STRUCTURE PROVIDING IMPROVED FIXATION AND EFFICIENT REMOVAL OF CORE FOR PREVENTING HYPOTONY**

(30) Priority: 22.01.2025 KR 20250009235
(71) Applicant: MICROT Inc., Seoul 06351 (KR)
(72) Inventor: LEE, Eun Suk, 13385 Gyeonggi-do (KR); LEE, Ye Ji, 25328 Gangwon-do (KR)
(74) Representative: Ter Meer Steinmeister & Partner

(57) **Abstract**

The disclosure provides an aqueous drainage implant structure providing improved fixation and efficient removal of a core for preventing hypotony, which is an implant structure used in minimally invasive glaucoma surgery (MIGS), the aqueous drainage implant structure including a tube unit having a hollow for providing a drainage path for aqueous humor, a wing unit provided to protrude from an outer surface of the tube unit to define a limit of insertion of the tube unit into a sclera, and a core unit inserted into the hollow to prevent a hypotony phenomenon that is able to occur after the MIGS.

## Description

This application claims priority of Korean Patent Application No. 10-2025-0009235, filed on January 22, 2025.

### BACKGROUND

### Field of the Invention

The disclosure relates to an aqueous drainage implant structure providing improved fixation and efficient removal of a core for preventing hypotony, and more particularly, to an aqueous drainage implant structure for ensuring completeness of surgery by preventing detachment of a core for preventing hypotony in an early stage of minimally invasive glaucoma surgery.

### Discussion of Related Art

Glaucoma is a representative ophthalmic disease that causes optic nerve damage and visual field loss, and it is known that the main cause is increased intraocular pressure due to abnormal drainage of aqueous humor.

The goal of glaucoma treatment is to effectively lower intraocular pressure, and drug treatment methods, laser treatment methods, surgical methods, etc., are used.

Here, drug treatment methods have problems that the treatment effect is limited if the patient's compliance is low, and side effects are likely to occur with long-term use, and laser treatment methods have problems that the effect is limited and repeat treatment is sometimes required.

Among surgical methods, trabeculectomy has a problem of a high risk of complications such as postoperative infection, scar formation, and excessive intraocular pressure reduction.

In order to overcome the limitations of the above conventional treatment or surgical methods, a minimally invasive glaucoma surgery (MIGS) method has recently been attracting attention as an alternative.

The MIGS method is a method of lowering intraocular pressure by inserting a small device that helps drain the aqueous humor, and has advantages that a recovery period is short and side effects are fewer through a minimally invasive approach.

A representative example of the above-mentioned small device is a microtube with a diameter of micrometers. One end of the microtube is inserted into the sclera to be positioned in the anterior chamber of the eyeball, and the other end is positioned in the subconjunctival space to enable stable drainage of the aqueous humor.

When performing MIGS using the microtube, initial postoperative hypotony should be prevented, and to this end, methods such as the XEN Glaucoma Treatment System have been used conventionally to reduce an inner diameter of the microtube.

However, the conventional methods of reducing the inner diameter of the microtube have a possibility that the postoperative intraocular pressure may not be lowered sufficiently and have a possibility that a drainage path may be blocked again, causing occlusive complications.

Therefore, there is an urgent need for research and development of a new microtube that can maximize the efficiency of aqueous humor drainage and provide stability and durability at the same time.

### SUMMARY OF THE INVENTION

The disclosure is directed to providing an aqueous drainage implant structure providing improved fixation and efficient removal of a core for preventing hypotony, which is an implant structure used in minimally invasive glaucoma surgery (MIGS), that can improve stability by preventing a hypotony phenomenon that may occur after the surgery, and at the same time, can improve the sustainability of effects of the surgery by preventing blockage of a drainage path.

The object is solved by the features of the independent claims. Preferred embodiments are given in the dependent claims.

According to an aspect of the disclosure, there is provided an aqueous drainage implant structure providing improved fixation and efficient removal of a core for preventing hypotony, which is an implant structure used in minimally invasive glaucoma surgery (MIGS), the aqueous drainage implant structure including a tube unit having a hollow for providing a drainage path for aqueous humor, a wing unit provided to protrude from an outer surface of the tube unit to define a limit of insertion of the tube unit into a sclera, and a core unit inserted into the hollow to prevent a hypotony phenomenon that is able to occur after the MIGS, wherein, for separation of the core unit from the tube unit to be prevented during the MIGS, the core unit has a separation preventing part provided on at least one of one exposed end and the other exposed end exposed outward from one end and the other end, respectively, of the tube unit, the one end inserted into the sclera and located in an anterior chamber of an eyeball.

In the aqueous drainage implant structure providing improved fixation and efficient removal of a core for preventing hypotony according to the disclosure, the separation preventing part may be provided on the other exposed end and may include a needle that enables stitching on the sclera through the other exposed end.

In the aqueous drainage implant structure providing improved fixation and efficient removal of a core for preventing hypotony according to the disclosure, on at least one of the one exposed end and the other exposed end, the separation preventing part may be provided with a size larger than the hollow and may limit a movement range of the core unit in the hollow.

In the aqueous drainage implant structure providing improved fixation and efficient removal of a core for preventing hypotony according to the disclosure, the separation preventing part may be elastically deformed and able to slide to the hollow so that the core unit is separated from the tube unit by an external force after a predetermined period of time since the MIGS.

In the aqueous drainage implant structure providing improved fixation and efficient removal of a core for preventing hypotony according to the disclosure, the separation preventing part may be made of a material that degrades within a predetermined period of time since the MIGS so that the core unit is separated from the tube unit by an external force after the predetermined period of time.

In the aqueous drainage implant structure providing improved fixation and efficient removal of a core for preventing hypotony according to the disclosure, the separation preventing part may be provided on the one exposed end and the other exposed end and may be provided to be longer than a length of the tube unit to have a separation distance therefrom.

In the aqueous drainage implant structure providing improved fixation and efficient removal of a core for preventing hypotony according to the disclosure, the separation preventing part may be provided in a state in which a tip of the one exposed end is rolled, may prevent the core unit from being separated from the tube unit during the MIGS, and may allow the core unit to slide to the hollow as the rolled state is released due to an external force after a predetermined period of time since the MIGS.

In the aqueous drainage implant structure providing improved fixation and efficient removal of a core for preventing hypotony according to the disclosure, the separation preventing part may be provided on a tip of the one exposed end where the one exposed end is bent toward the one end, may be caught at an outer surface of the one end during the MIGS, and may allow the core unit to slide to the hollow as the caught state is released due to an external force after a predetermined period of time since the MIGS.

The aqueous drainage implant structure providing improved fixation and efficient removal of a core for preventing hypotony according to the disclosure may further include a socket unit inserted into an outer surface of the one end so that the tip disposed on the outer surface of the one end is pressed.

In the aqueous drainage implant structure providing improved fixation and efficient removal of a core for preventing hypotony according to the disclosure, the separation preventing part may be provided on a tip of the one exposed end where the one exposed end is bent toward the one end and inserted into the hollow, and the aqueous drainage implant structure may further include a socket unit inserted into the hollow so that the tip disposed in the hollow is pressed.

In the aqueous drainage implant structure providing improved fixation and efficient removal of a core for preventing hypotony according to the disclosure, the separation preventing part may be provided on a branched end branched from a specific point of the one exposed end toward the one end, may be caught at an outer surface of the one end during the MIGS, and may allow the core unit to slide to the hollow as the caught state is released due to an external force after a predetermined period of time since the MIGS.

### BRIEF DESCRIPTION OF THE DRAWINGS

The above and other objects, features, and advantages of the disclosure will become more apparent to those of ordinary skill in the art by describing exemplary embodiments thereof in detail with reference to the accompanying drawings, in which:
FIG. 1 is a view for describing a state in which, by minimally invasive glaucoma surgery (MIGS), an aqueous drainage implant structure providing improved fixation and efficient removal of a core for preventing hypotony according to the disclosure is inserted into an eyeball;
FIG. 2 is a view illustrating an aqueous drainage implant structure providing improved fixation and efficient removal of a core for preventing hypotony of a first embodiment of the disclosure;
FIG. 3 a view of an aqueous drainage implant structure providing improved fixation and efficient removal of a core for preventing hypotony of a second embodiment of the disclosure;
FIG. 4 is a view illustrating an aqueous drainage implant structure providing improved fixation and efficient removal of a core for preventing hypotony of a third embodiment of the disclosure;
FIG. 5 is a view of an aqueous drainage implant structure providing improved fixation and efficient removal of a core for preventing hypotony of a fourth embodiment of the disclosure;
FIG. 6 is a view illustrating an aqueous drainage implant structure providing improved fixation and efficient removal of a core for preventing hypotony of a fifth embodiment of the disclosure;
FIG. 7 is a view illustrating an aqueous drainage implant structure providing improved fixation and efficient removal of a core for preventing hypotony of a sixth embodiment of the disclosure;
FIG. 8 is a view of an aqueous drainage implant structure providing improved fixation and efficient removal of a core for preventing hypotony of a seventh embodiment of the disclosure;
FIG. 9 is a view of an aqueous drainage implant structure providing improved fixation and efficient removal of a core for preventing hypotony of an eighth embodiment of the disclosure;
FIG. 10 is a view of an aqueous drainage implant structure providing improved fixation and efficient removal of a core for preventing hypotony of a ninth embodiment of the disclosure.

### DETAILED DESCRIPTION OF EXEMPLARY EMBODIMENTS

Hereinafter, specific embodiments of the disclosure will be described in detail with reference to the accompanying drawings. However, the concept of the disclosure is not limited by the embodiments ed herein, and those of ordinary skill in the art who understand the concept of the disclosure may easily propose other less advanced inventions or other embodiments belonging to the scope of the concept of the disclosure by adding another component, changing a component, or omitting a component within the scope of the same concept, but such inventions or embodiments also pertain to the scope of the concept of the disclosure.

In addition, components with the same function within the scope of the same concept shown in the drawings of different embodiments will be described using the same reference numerals.

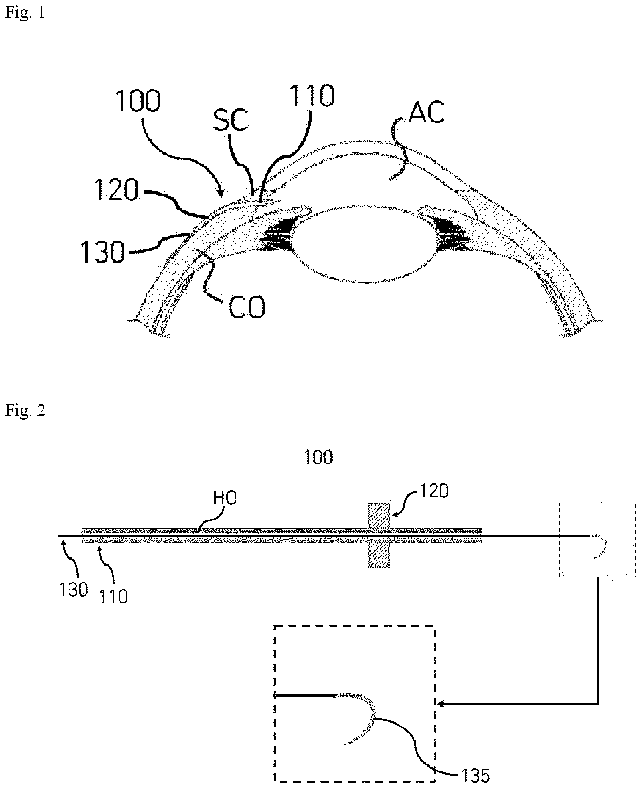

FIG. 1 is a view for describing a state in which, by minimally invasive glaucoma surgery (MIGS), an aqueous drainage implant structure providing improved fixation and efficient removal of a core for preventing hypotony according to the disclosure is inserted into an eyeball.

Referring to FIG. 1, an aqueous drainage implant structure 100 providing improved fixation and efficient removal of a core for preventing hypotony according to the disclosure (hereinafter referred to as "implant structure 100") may be a type of microtube with a diameter of micrometers that is used in MIGS to help drain aqueous humor and prevent an increase in intraocular pressure.

One end of the implant structure 100 may be inserted into a sclera SC, which is exposed when scleral flaps are cut open through an incision, and located in an anterior chamber AC, and the other end of the implant structure 100 may be located on the exposed sclera SC (located in the subconjunctival space) and may enable stable drainage of aqueous humor from the anterior chamber to the episcleral space (subconjunctival space), thereby preventing an excessive increase in intraocular pressure in the eyeball.

The implant structure 100 may include a tube unit 110 for providing a drainage path for aqueous humor, a wing unit 120 for defining a limit of insertion of the tube unit 110 into the sclera SC, and a core unit 130 for preventing a hypotony phenomenon that may occur after the surgery, and these will be described in detail below.

FIG. 2 is a view illustrating an aqueous drainage implant structure providing improved fixation and efficient removal of a core for preventing hypotony according to a first embodiment of the disclosure.

Referring to FIG. 2, an implant structure 100 according to a first embodiment of the disclosure may include a tube unit 110 having a hollow HO for providing a drainage path for aqueous humor, a wing unit 120 provided to protrude from an outer surface of the tube unit 110 to define a limit of insertion of the tube unit 110 into the sclera, and a core unit 130 inserted into the hollow to prevent a hypotony phenomenon that may occur after the MIGS.

The tube unit 110 may be made of a material with a surface that is smooth enough to not affect the flow of aqueous humor and may be made of a biocompatible material.

For example, the tube unit 110 may be made of a silicone-based material, polytetrafluoroethylene (PTFE), polycarbonate (PC), a urethane-based material such as polyurethane (PU), a compound of a silicone-based material and a PU-based material such as silicone-PU, or a biocompatible metal or alloy.

In addition, the tube unit 110 may be made of any one of silicone, PTFE, PC, PU, polyethylene, polypropylene, polyimide, polymethyl methacrylate (PMMA), a poly(styrene-b-isobutylene-b-styrene) copolymer, polyethersulfone, gelatin, stainless steel, titanium, and nitinol or a combination of one or more thereof.

The tube unit 110 may be made with an inner diameter ranging from 95 µm to 105 µm, an outer diameter ranging from 216.6 µm to 239.4 µm, and a length ranging from 5.7 mm to 6.2 mm but is not necessarily limited thereto.

The tube unit 110 is provided with a relatively greater inner diameter compared to the conventional XEN Glaucoma Treatment System, and thus compared to the related art, the tube unit 110 can facilitate drainage of aqueous humor for lowering the intraocular pressure and can maximize the sustainability of effects of the surgery (preventing occlusive complications) by significantly decreasing the possibility of blockage of a drainage path for aqueous humor.

The wing unit 120 may be made of the same biocompatible material as the tube unit 110 and may be provided to protrude at a predetermined angle (for example, in a perpendicular direction) based on the longitudinal direction of the tube unit 110.

The wing unit 120 provides an effect of causing the tube unit 110 corresponding to a front end of the wing unit 120 to be caught at the sclera in a process of being inserted through the sclera, and as a result, the tube unit 110 corresponding to a rear end of the wing unit 120 can be prevented from being inserted into the sclera.

The wing unit 120 may be formed by being inserted into an outer surface of the tube unit 110 and then being joined or adhered thereto, but the disclosure is not necessarily limited thereto, and the wing unit 120 may be integrally formed with the tube unit 110.

The core unit 130 may be made of a material capable of maintaining its shape without deformation even when in contact with aqueous humor and may be a unit inserted into the hollow to prevent a hypotony phenomenon, which is caused by sudden drainage of a large amount of aqueous humor after the MIGS, to ensure positive effects of the tube unit 110 being formed with a relatively greater inner diameter compared to the related art.

The core unit 130 may be a non-absorbable surgical suture for surgical use, and for example, may be made of materials such as nylon or prolene.

The core unit 130 may be removed after a period of time after which no further hypotony problems are considered to occur since the MIGS, e.g., 4 weeks to 16 weeks.

Meanwhile, for separation of the core unit 130 from the tube unit 110 to be prevented during the MIGS, the core unit 130 may have a separation preventing part 135 provided on at least one of one exposed end and the other exposed end exposed outward from one end and the other end, respectively, of the tube unit 110.

Here, the one end may be a portion inserted into the sclera, which is exposed when scleral flaps are cut open through an incision, and located in the anterior chamber of the eyeball, and the other end may be a portion exposed in the space on the exposed sclera SC (subconjunctival space).

In the case of a core unit without the separation preventing part 135, due to a difference between an outer diameter of the core unit and an inner diameter of the tube unit, there is a high possibility that the core unit will be separated from the hollow of the tube unit even when a slight external force is applied, and in this case, a cumbersome situation may occur in which a surgeon must pick up the core unit with medical forceps or the like and insert it back into the hollow.

Therefore, in the disclosure, by the separation preventing part 135 preventing separation of the core unit 130 from the tube unit 110 during the surgery, convenience can be provided to the surgeon performing the surgery, and stability of the surgery can be ensured.

Specifically, the separation preventing part 135 may be provided on the other exposed end and may include a needle 135 that enables stitching on the sclera through the other exposed end.

The other exposed end of the core unit 130 may itself serve as a type of suture through the needle 135 and may be stitched on the sclera, and as a result, the core unit 130 is prevented from deviating from the tube unit 110 during the MIGS.

The MIGS includes multiple steps such as incision of scleral flaps, insertion of the implant structure 100, and suturing, and in this process, an effect of preventing separation of the core unit 130 from the tube unit 110 by the separation preventing part 135 allows the surgery to be stably performed, thereby ensuring completeness of the surgery.

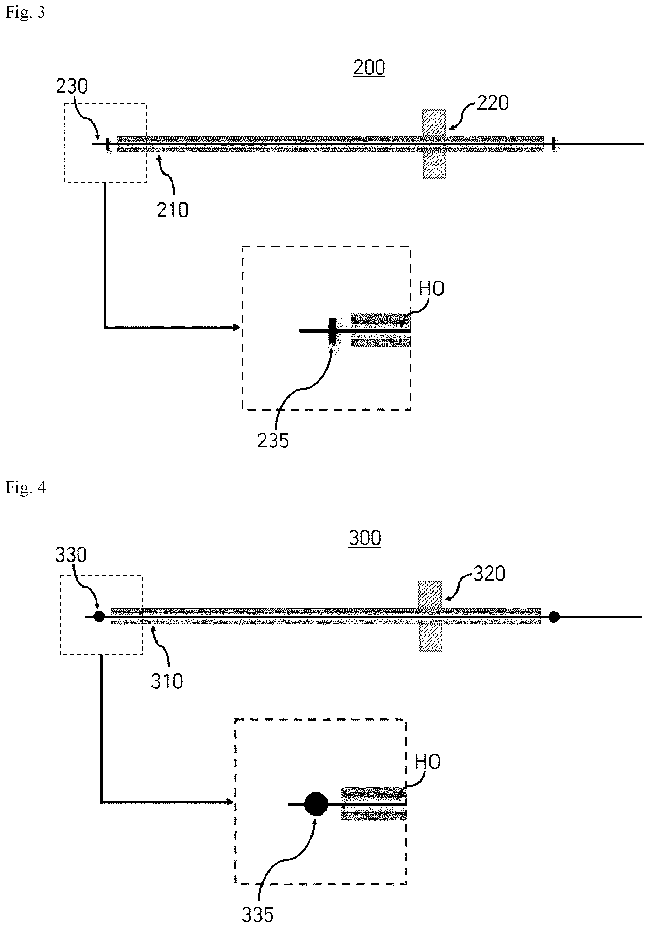

FIG. 3 is a view illustrating an aqueous drainage implant structure providing improved fixation and efficient removal of a core for preventing hypotony according to a second embodiment of the disclosure, and FIG. 4 is a view illustrating an aqueous drainage implant structure providing improved fixation and efficient removal of a core for preventing hypotony according to a third embodiment of the disclosure.

Referring to FIGS. 3 and 4, implant structures 200 and 300 according to the second and third embodiments of the disclosure may include tube units 210 and 310, wing units 220 and 320, and core units 230 and 330, and the core units 230 and 330 may include separation preventing parts 235 and 335.

On at least one of one exposed end and the other exposed end of the core units 230 and 330, the separation preventing parts 235 and 335 may be provided with a size larger than a hollow HO of the tube units 210 and 310 and may limit a movement range of the core units 230 and 330 in the hollow HO.

Since limiting the movement range of the core units 230 and 330 has substantially the same meaning as preventing the core units 230 and 330 from deviating from the tube units 210 and 310 during the MIGS, the surgery can be stably performed, and the completeness of the surgery can be ensured.

The separation preventing parts 235 and 335 may be provided on the one exposed end and the other exposed end and may be provided to be longer than lengths of the tube units 210 and 310 to have a separation distance therefrom.

The separation distance may ensure movability of the core units 230 and 330 relative to the tube units 210 and 310 and may prevent a phenomenon of blockage of the hollow HO providing a drainage path for aqueous humor, and from the perspective of the surgeon performing the surgery, it becomes easy to control the core units 230 and 330 based on the tube units 210 and 310.

The separation preventing part 235 illustrated in FIG. 3 may be elastically deformed and able to slide to the hollow HO so that the core unit 230 is separated from the tube unit 210 by an external force after a predetermined period of time since the MIGS.

The separation preventing part 335 illustrated in FIG. 4 may be made of a material that degrades within a predetermined period of time since the MIGS so that the core unit 330 is separated from the tube unit 310 by an external force after the predetermined period of time.

Here, the predetermined period of time is a period of time after which no further hypotony problems are considered to occur since the MIGS, e.g., 4 weeks to 16 weeks, and is the same as that described above with reference to FIG. 2.

The degrading material is a biodegradable component and may include any known biodegradable component such as polyglycolic acid (PGA), polylactic acid (PLA), polydioxanone (PDO), copolymers of glycolide and lactide, and catgut.

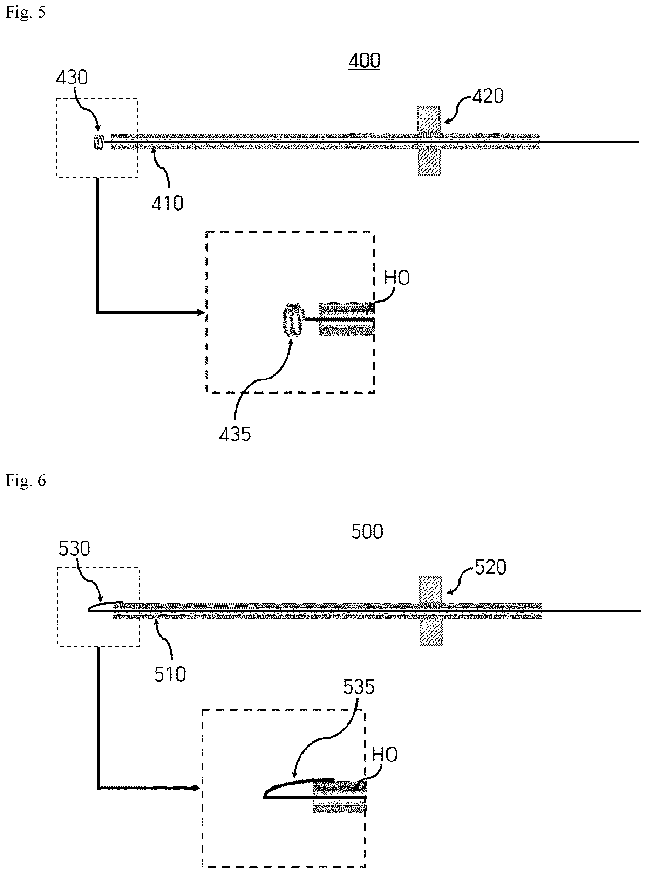

FIG. 5 is a view illustrating an aqueous drainage implant structure providing improved fixation and efficient removal of a core for preventing hypotony according to a fourth embodiment of the disclosure.

Referring to FIG. 5, an implant structure 400 according to the fourth embodiment may include a tube unit 410, a wing unit 420, and a core unit 430, and the core unit 430 may include a separation preventing part 435.

The separation preventing part 435 may be provided in a state in which a tip of one exposed end of the core unit 430 is rolled, may prevent the core unit 430 from being separated from the tube unit 410 during the MIGS, and may allow the core unit 430 to slide to a hollow HO as the rolled state is released due to an external force after a predetermined period of time since the MIGS.

That is, the separation preventing part 435 may prevent the core unit 430 from being separated from the tube unit 410 by an external force generated during the surgery but may allow the core unit 430 to be separated from the tube unit 410 as the rolled state is released due to an artificial external force for removing the core unit 430 after the predetermined period of time, that is, a period of time after which no further hypotony problems are considered to occur since the surgery.

FIG. 6 is a view illustrating an aqueous drainage implant structure providing improved fixation and efficient removal of a core for preventing hypotony according to a fifth embodiment of the disclosure.

Referring to FIG. 6, an implant structure 500 according to the fifth embodiment of the disclosure may include a tube unit 510, a wing unit 520, and a core unit 530, and the core unit 530 may include a separation preventing part 535.

The separation preventing part 535 may be provided on a tip of one exposed end of the core unit 530 where the one exposed end is bent toward one end of the tube unit 510.

The separation preventing part 535 may be caught at an outer surface of the one end during the MIGS and may allow the core unit 530 to slide to a hollow HO as the caught state is released due to an external force after a predetermined period of time since the MIGS.

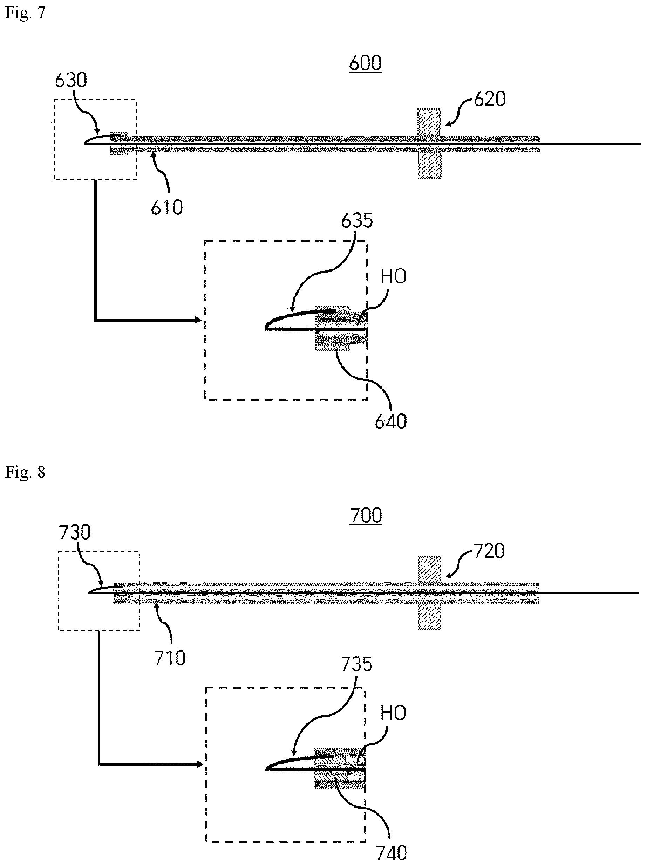

FIG. 7 is a view illustrating an aqueous drainage implant structure providing improved fixation and efficient removal of a core for preventing hypotony according to a sixth embodiment of the disclosure.

Referring to FIG. 7, since an implant structure 600 according to the sixth embodiment of the disclosure has the same configurations and effects as the implant structure 500 according to the fifth embodiment of the disclosure described above with reference to FIG. 6 except for a socket unit 640, descriptions of components other than the socket unit 640 will be omitted.

The socket unit 640 may be a cylindrical unit inserted into an outer surface of one end of a tube unit 610 so that a tip of a core unit 630 disposed on the outer surface of the one end is pressed.

The socket unit 640 may have an inner diameter that allows insertion into the tube unit 610 and may have an outer diameter ranging from 240 µm to 340 µm.

The socket unit 640 may be made of the same material as the tube unit 610 and may allow separation of the core unit 630 from the tube unit 610 to be more effectively prevented during the MIGS compared to the fifth embodiment described above with reference to FIG. 6.

Meanwhile, due to the socket unit 640, a magnitude of an external force for removing the core unit 630 after the MIGS may be somewhat greater than the magnitude of the external force according to the fifth embodiment.

FIG. 8 is a view illustrating an aqueous drainage implant structure providing improved fixation and efficient removal of a core for preventing hypotony according to a seventh embodiment of the disclosure.

Referring to FIG. 8, an implant structure 700 according to the seventh embodiment of the disclosure may include a tube unit 710, a wing unit 720, a core unit 730, and a socket unit 740, and the core unit 730 may include a separation preventing part 735.

The separation preventing part 735 may be provided on a tip of one exposed end of the core unit 730 where the one exposed end is bent toward one end of the tube unit 710 and inserted into a hollow HO.

Here, the socket unit 740 may be made of the same material as the tube unit 710 and may be a cylindrical unit inserted into the hollow HO so that the tip disposed in the hollow HO is pressed.

The socket unit 740 may have an outer diameter that allows insertion into the hollow HO and may have an inner diameter in a range in which drainage of aqueous humor is possible.

The socket unit 740 may, due to pressing the tip, more effectively prevent separation of the core unit 730 from the tube unit 710 during MIGS, and a magnitude of an external force for removing the core unit 730 after the MIGS may be somewhat greater.

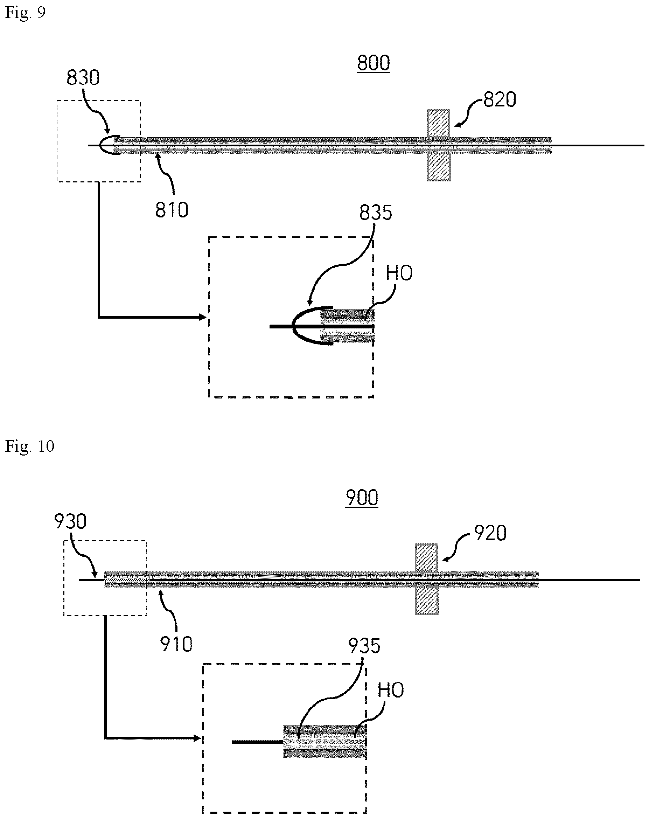

FIG. 9 is a view illustrating an aqueous drainage implant structure providing improved fixation and efficient removal of a core for preventing hypotony according to an eighth embodiment of the disclosure.

Referring to FIG. 9, an implant structure 800 according to the eighth embodiment of the disclosure may include a tube unit 810, a wing unit 820, and a core unit 830, and the core unit 830 may include a separation preventing part 835.

The separation preventing part 835 may be provided on a branched end branched from a specific point of one exposed end of the core unit 830 toward one end of the tube unit 810.

The branched end may be caught at an outer surface of the one end during the MIGS and may allow the core unit 830 to slide to a hollow HO as the caught state is released due to an external force after a predetermined period of time since the MIGS.

FIG. 10 is a view illustrating an aqueous drainage implant structure providing improved fixation and efficient removal of a core for preventing hypotony according to a ninth embodiment of the disclosure.

Referring to FIG. 10, an implant structure 900 according to the ninth embodiment of the disclosure may include a tube unit 910, a wing unit 920, and a core unit 930, and the core unit 930 may include a separation preventing part 935.

The separation preventing part 935 may be provided as a fine protrusion on a predetermined portion disposed in a hollow HO in one end of the tube unit 910 so that separation from the tube unit 910 is prevented during MIGS.

Here, the one end is a portion inserted into the sclera and located in the anterior chamber of the eyeball, and the fine protrusion may generate a resistance force during the surgery and may more effectively prevent separation of the core unit 930 from the tube unit 910.

According to an aqueous drainage implant structure providing improved fixation and efficient removal of a core for preventing hypotony according to the disclosure, stability of glaucoma surgery can be ensured by a core unit preventing a hypotony phenomenon that may occur after minimally invasive glaucoma surgery (MIGS).

In addition, it is possible to provide convenience to a surgeon performing the surgery by making it possible to stably fix the position of the core unit during the MIGS.

Further, it is possible to improve the sustainability of effects of intraocular pressure control by securing the size of a hollow, which is a movement path for aqueous humor, and preventing blockage of a drainage path after the surgery.

Configurations and features of the disclosure have been described above based on embodiments according to the disclosure, but the disclosure is not limited thereto. It should be apparent to those of ordinary skill in the art to which the disclosure pertains that various changes or modifications may be made within the concept and scope of the disclosure, and it should be noted that such changes or modifications also fall within the scope of the appended claims.

## Claims

1. An aqueous drainage implant structure providing improved fixation and efficient removal of a core for preventing hypotony that is used in minimally invasive glaucoma surgery (MIGS), the aqueous drainage implant structure comprising:
a tube unit (110) having a hollow (HO) for providing a drainage path for aqueous humor;
a wing unit (120) provided to protrude from an outer surface of the tube unit (110) to define a limit of insertion of the tube unit (110) into a sclera (SC); and
a core unit (130) inserted into the hollow (HO) to prevent a hypotony phenomenon that is able to occur after the MIGS,
wherein, for separation of the core unit (130) from the tube unit (110) to be prevented during the MIGS, the core unit (130) has a separation preventing part (135) provided on at least one of one exposed end and the other exposed end exposed outward from one end and the other end, respectively, of the tube unit (110), the one end inserted into the sclera (SC) and located in an anterior chamber of an eyeball.

2. The aqueous drainage implant structure of claim 1, wherein the separation preventing part (135) is provided on the other exposed end and includes a needle (135) that enables stitching on the sclera (SC) through the other exposed end.

3. The aqueous drainage implant structure of claim 1 or 2, wherein, on at least one of the one exposed end and the other exposed end, the separation preventing part (135) is provided with a size larger than the hollow (HO) and limits a movement range of the core unit (130) in the hollow (HO).

4. The aqueous drainage implant structure of any one of the preceding claims, wherein the separation preventing part (135) is elastically deformed and able to slide to the hollow (HO) so that the core unit (130) is separated from the tube unit (110) by an external force after a predetermined period of time since the MIGS.

5. The aqueous drainage implant structure of any one of the preceding claims, wherein the separation preventing part (135) is made of a material that degrades within a predetermined period of time since the MIGS so that the core unit (130) is separated from the tube unit (110) by an external force after the predetermined period of time.

6. The aqueous drainage implant structure of any one of the preceding claims, wherein the separation preventing part (135) is provided on the one exposed end and the other exposed end and is provided to be longer than a length of the tube unit (110) to have a separation distance therefrom.

7. The aqueous drainage implant structure of any one of the preceding claims, wherein the separation preventing part (135) is provided in a state in which a tip of the one exposed end is rolled.

8. The aqueous drainage implant structure of any one of the preceding claims, wherein the separation preventing part (135) prevents the core unit (110) from being separated from the tube unit (110) during the MIGS, and/or allows the core unit (130) to slide to the hollow (HO) as the rolled state is released due to an external force after a predetermined period of time since the MIGS.

9. The aqueous drainage implant structure of any one of the preceding claims, wherein the separation preventing part (135) is provided on a tip of the one exposed end where the one exposed end is bent toward the one end.

10. The aqueous drainage implant structure of any one of the preceding claims, wherein the separation preventing part (135) is caught at an outer surface of the one end during the MIGS, and allows the core unit (130) to slide to the hollow as the caught state is released due to an external force after a predetermined period of time since the MIGS.

11. The aqueous drainage implant structure of claim 9 or 10, further comprising a socket unit (640) inserted into an outer surface of the one end so that the tip disposed on the outer surface of the one end is pressed.

12. The aqueous drainage implant structure of any one of the preceding claims, wherein:
the separation preventing part (135) is provided on a tip of the one exposed end where the one exposed end is bent toward the one end and inserted into the hollow (HO); and
the aqueous drainage implant structure further comprises a socket unit (640) inserted into the hollow (HO) so that the tip disposed in the hollow (HO) is pressed.

13. The aqueous drainage implant structure of any one of the preceding claims, wherein the separation preventing part (135) is provided on a branched end branched from a specific point of the one exposed end toward the one end.

14. The aqueous drainage implant structure of any one of the preceding claims, wherein the separation preventing part (135) is caught at an outer surface of the one end during the MIGS, and/or allows the core unit (135) to slide to the hollow (HO) as the caught state is released due to an external force after a predetermined period of time since the MIGS.
